(19) Europäisches Patentamt
European Patent Office
Office européen des brevets

(11) Publication number: **0 357 792 B1**

(12) **EUROPEAN PATENT SPECIFICATION**

(45) Date of publication of patent specification: **28.06.95**

(51) Int. Cl.[6]: **C07D 213/803**

(21) Application number: **89903222.1**

(22) Date of filing: **17.03.89**

(86) International application number:
**PCT/JP89/00288**

(87) International publication number:
**WO 89/08645 (21.09.89 89/23)**

Divisional application 93116799.3 filed on 17/03/89.

The file contains technical information submitted after the application was filed and not included in this specification

# (54) PROCESS FOR PREPARING PYRIDINE-2,3-DICARBOXYLIC ACID COMPOUNDS.

(30) Priority: **18.03.88 JP 66772/88**

(43) Date of publication of application:
**14.03.90 Bulletin 90/11**

(45) Publication of the grant of the patent:
**28.06.95 Bulletin 95/26**

(84) Designated Contracting States:
**BE CH DE FR GB IT LI NL**

(56) References cited:
**EP-A- 0 220 518**
**EP-A- 0 274 379**

**THE JOURNAL OF THE AMERICAN SOCIETY, vol. 72, September-December 150, pages 5221-5225; L.H. CONOVER et al.: "Thiazole analogs of pyridoxine"**

(73) Proprietor: **SUGAI CHEMICAL INDUSTRY CO., LTD.**
**4-6, 4-chome, Uzu**
**Wakayama-shi**
**Wakayama-ken (JP)**

(72) Inventor: **YAMASHITA, Takaharu**
**Sugai Chem Ind Co.Ltd 4-6,Uzu 4-chome**
**Wakayama-shi Wakayama 641 (JP)**
Inventor: **KODAMA, Mitsuhiro**
**Sugai Chem Ind Co. Ltd 4-6, Uzu 4-chome**
**Wakayama-shi Wakayama 641 (JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 357 792 B1

JOURNAL OF THE CHEMICAL SOCIETY, vol. 438, part III, 1949, pages 2049-2054, TheChemical Society, London, GB; G.W. HOLTON et al.: "Experiments on the synthesis

of rotenone and its derivatives. part XVI. The synthesis of abutic acid and itsanalogues"

(74) Representative: **Hansen, Bernd, Dr.rer.nat. et al**
**Hoffmann, Eitle & Partner**
**Patentanwälte**
**Postfach 81 04 20**
**D-81904 München (DE)**

## Description

### Technical Field

This invention relates to a process for preparing pyridine-2,3-dicarboxylic acid compounds. More particularly, it relates to a process for preparing pyridine-2,3-dicarboxylic acid compounds which are useful as an intermediate for manufacturing agricultural chemicals and pharmaceuticals.

### Background Art

Heretofore, as the method of preparing pyridine-2, 3-dicarboxylic acid compounds known are:

(1) Oxidation with nitric acid of quinolines and quinolinols which are synthesized by Skraup reaction from aniline and glycerine with concentrated sulfuric acid and nitrobenzene. (J. Chem. Soc. page 4433, 1956);

(2) Reacting an $\alpha,\beta$-unsaturated hydrazone compound and a maleic acid compound in an inert solvent to obtain 1-(substituted amino)-1,4-dihydropyridine-2,3-dicarboxylic acid derivative. Then, heating the resultant derivative to eliminate the substituted amino group in the 1-position. (Japanese Patent Application Unexamined Publication No. 246369/1985).

(3) Treating a 1-(substituted amino)-1,2,3,4-tetrahydropyridine-2,3-dicarboxylic acid derivative with acid and/or by heat to convert into a 1,4-dihydropyridine-2,3-dicarboxylic acid derivative, and then oxidizing it. (Japanese Patent Application Unexamined Publication No. 47482/1986).

(4) Oxidizing quinoline with excess hypochlorites in the presence of ruthenium oxide. (Japanese Patent Application Unexamined Publication No. 212563/1986).

(5) Condensing and cyclizing $\alpha$-halo-$\beta$-keto-esters and $\alpha,\beta$-unsaturated aldehydes or ketones in the organic solvent in the presence of more than 2 mole equivalent of ammonium salt. (Japanese Patent Application Unexamined Publication No. 106081/1987).

As the method of synthesizing pyridinemonocarboxylic acid derivatives known is such a method as subjecting ethyl 2-methly-1,4-dihydronicotinate which is produced by condensation and cyclization of $\alpha,\beta$-unsaturated aldehydes such as acrolein and crotonaldehyde with ethyl $\beta$-aminocrotonate to oxidation with nitric acid in a mixed acid. (J. Org. Chem. Soc. Vol. 21, page 800, 1956).

However, the above method (1) not only has many reaction processes but also requires drastic oxidation with nitric acid, and involves possible hazards. Also, the pyridine-2,3-dicarboxylic acids, which are apt to cause decarboxylation, result in low yields by the oxidation with nitric acid, and, in addition, produce a large quantity of acidic waste liquid. Thus, the method (1) is not suited to the industrial manufacture of pyridine-2,3-dicarboxylic acids.

The methods (2) and (3) mentioned above have many reaction processes leading to decreased total yields, and require the use of expensive starting materials. Particularly, they require elimination process of the substituted amino group in the intermediate. This decreases the yield and becomes a problem on resource saving. Therefore, it is difficult to manufacture pyridine-2,3-dicarboxylic acid derivatives industrially by the method (2) or (3).

In the method (4), there are problems that a large excess of the oxidant must be used and that a large quantity of waste liquid is produced requiring expenses for its disposal.

In the method (5), $\alpha$-halo-$\beta$-keto-ester used as a raw material cannot be produced in good yield with conventional known manufacturing methods, causing high cost of raw material. It is, therefore, difficult to manufacture pyridine-2,3-dicarboxylic acid derivatives industrially by the method (5).

From J. Am. Chem. Soc. 1950, 72, 5521-5 it has been known to react an alkali metal salt of oxaloacetic acid in chloroform with sulfuryl chloride, to give an $\alpha$-halooxaloacetate. However, the yield of the end product is not high, i.e. about 30%.

Accordingly, it is an object of this invention to provide a process for preparing pyridine-2,3-dicarboxylic acid compounds in high yield from starting materials inexpensive and readily available.

### Disclosure of Invention

A process for preparing pyridine-2,3-dicarboxylic acid compounds of the invention is a process for preparing pyridine-2,3-dicarboxylic acid compounds represented by the following formula (1).

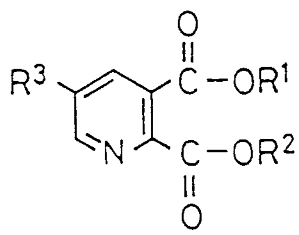

(1)

wherein $R^1$ and $R^2$ are, identical or different, a lower alkyl group, and $R^3$ is a hydrogen atom or a lower alkyl group,
which comprises reacting
a compound of the following formula (2):

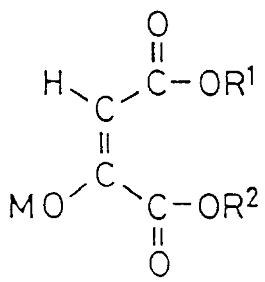

(2)

wherein $R^1$ and $R^2$ are the same as defined above and M is an alkali metal,
with an acid and a halogenating agent to give a compound of the following formula (3):

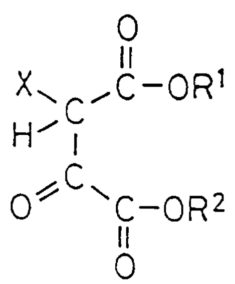

(3)

wherein $R^1$ and $R^2$ are the same as defined above and X is a halogen atom, and
then reacting the compound of the above formula (3) with the compound of the following formula (4):

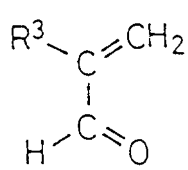

(4)

wherein $R^3$ is the same as defined above,
and ammonia, wherein the compound of formula (3) resulting from the reaction of the compound of formula (2) with an acid and a halogenating agent is reacted without isolation with the compound of formula (4) and ammonia.

EP-A-0 274 379 (document according to Art. 54(3) EPC) discloses a process for preparing pyridin-2,3-dicarboxylic acid compounds by reacting the compound having the above mentioned formula (3) with a compound having the above formula (4) and ammonia.

However, this document does not contain any disclosure as to the specific preparation of the compound having the formula (3) and to the use of this compound without isolation during the further reaction.

In each formula mentioned above, examples of the lower alkyl groups for $R^1$, $R^2$, and $R^3$ include, for example, straight or branched chain alkyl groups having 1 to 8 carbon atoms such as methyl, ethyl, propyl, isopropyl, butyl, isobutyl, tertiary-butyl, pentyl, hexyl, heptyl, octyl, 2-ethylhexyl group, and the like.

Examples of alkali metal for M include, for example, sodium, potassium, and the like.

Examples of the halogen atom for X include, for example, fluorine atom, chlorine atom, bromine atom, and the like.

Examples of the compound of the formula (2) include, for example, dimethyl oxalacetate sodium salt, diethyl oxalacetate sodium salt, methyl ethyl oxalacetate sodium salt, dimethyl oxalacetate potassium salt, diethyl oxalacetate potassium salt, di-tertiary-butyl oxalacetate sodium salt, di-tertiary-butyl oxalacetate potassium salt, dipropyl oxalacetate sodium salt, dipropyl oxalacetate potassium salt, and the like.

Examples of the compound of the formula (3) include, for example, dimethyl α-chlorooxalacetate, diethyl α-chlorooxalacetate, methyl ethyl α-chlorooxalacetate, dimethyl α-bromooxalacetate, diethyl α-bromooxalacetate, di-tertiary-butyl α-chlorooxalacetate, dipropyl α-chlorooxalacetate, dipropyl α-fluorooxalacetate, and the like.

Examples of the compound of the formula (4) include, for example, acrolein, 2-methyl-2-propenal, 2-ethyl-2-propenal, 2-propyl-2-propenal, 2-isopropyl-2-propenal, 2-butyl-2-propenal, 2-pentyl-2-propenal, 2-hexyl-2-propenal, 2-heptyl-2-propenal, 2-octyl-2-propenal, and the like.

The preparation method of this invention can be shown with the following reaction scheme 1.

Reaction scheme 1

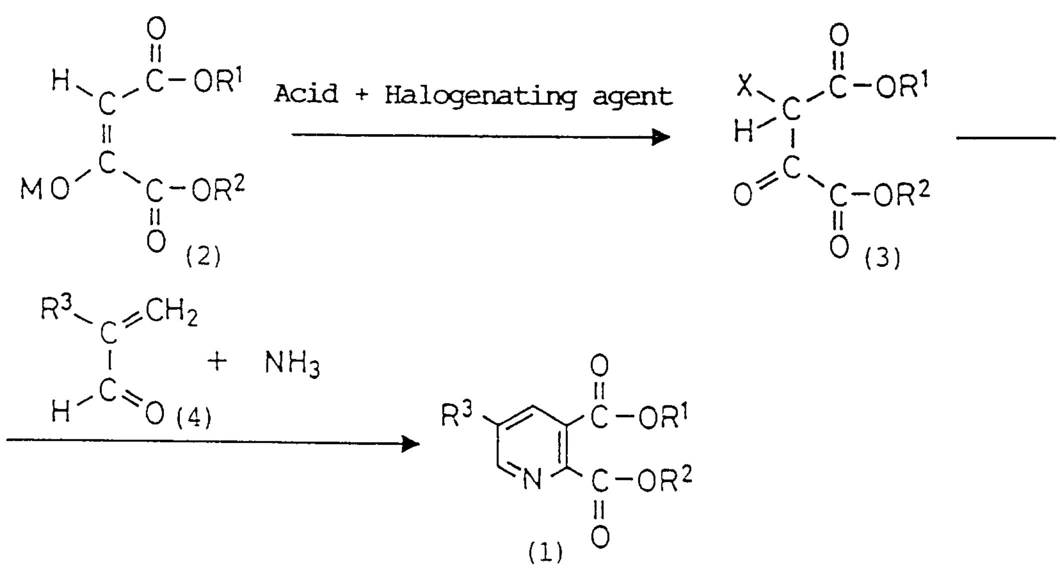


In the above formulae, $R^1$, $R^2$, $R^3$, X, and M are the same as defined before.

In the above Reaction scheme , the reaction of the reaction scheme 1 comprises a step which obtains the compound of the formula (3) by reacting the compound of the formula (2), an acid and a halogenating agent (step 1), and a step which obtains the compound of the formula (1) by reacting the compound of the formula (3) obtained above, the compound of the formula (4) and ammonia (step 2).

In the reaction of the step 1, examples of the acid used in the step include inorganic acids such as hydrochloric acid, sulfuric acid, nitric acid, and phosphoric acid, and organic acids including lower alkanoic acids such as formic acid, oxalic acid, acetic acid, and propionic acid, and sulfonic acids such as methanesulfonic acid, ethanesulfonic acid, benzenesulfonic acid, and toluenesulfonic acid. Examples of the halogenating agent include sulfuryl chloride, bromine, and chlorine.

This reaction can be carried out by either process, in which the compound of the formula (2) is allowed to react with the halogenating agent in the presence of the acid or in which the compound of the formula (2) is treated with the acid and then allowed to react with the halogenating agent .

It is desirable to carry out the reaction in an organic solvent. Any organic solvent may be used unless the solvent affects the reaction, but aprotic solvents including aromatic hydrocarbons such as toluene and benzene, and halogenated hydrocarbons such as chloroform, 1,2-dichloroethane and carbon tetrachloride are desirable.

The reaction temperature at which reaction of the compound of the formula (2) or its acid-treated compound with the halogenating agent takes place is not particularly specified, but, in general, the reaction is carried out at 0 - 100 °C, and preferably at 1 - 50 °C. The reaction is completed in 30 minutes to 10 hours, generally 1 to 5 hours.

The molar ratio of compound of the formula (2) to the halogenating agent can be varied widely, but in general it is desirable to use 0.6 - 1.5 mols, preferably 1 - 1.3 mols of the halogenating agent to 1 mol of the compound of the formula (2). The acid is used at least an equimolar amount, and preferably slightly in excess to the compound of the formula (2).

Combinations of the above-mentioned acid, organic solvent, and halogenating agent are optional, but it is most desirable to combine alkanoic acids, in particular, formic acid as acid, and halogenated hydrocarbons, in particular, chloroform as organic solvent, and sulfuryl chloride as halogenating agent.

The compound of the formula (3) which is obtained in this way can be used for the reaction of step 2 without isolation or after isolation and purification by a manner such as distillation and column chromatography.

The reaction of the step 2 is carried out to obtain the compound of the formula (1) by allowing the compound of the formula (3) obtained above to react with the compound of the formula (4) and ammonia.

The reaction is carried out under the condition free from solvent or in the organic solvent. Any organic solvent which does not affect the reaction can be used irrespective of polar, nonpolar, protic, or aprotic. Examples of solvents include alcohol such as methanol, ethanol, isopropyl alcohol, and butanol; halogenated hydrocarbons such as chloroform, 1,2-dichloroethane, and carbon tetrachloride; aromatic hydrocarbons such as benzene, toluene, xylene, chlorobenzene, o-dichlorobenzene, and nitrobenzene; ethers such as dimethyl ether, diethyl ether, dibutyl ether, tetrahydrofuran, dioxane, ethylene glycol dimethyl ether, ethylene glycol diethyl ether, diethylene glycol dimethyl ether, and dibenzyl ether; esters such as methyl acetate and ethyl acetate; aprotic polar solvents, for example, sulfoxides such as dimetyl sulfoxide, carboamides such as N,N-dimethylformamide, sulfones such as dimethyl sulfone and sulfolane, and hexamethylphosphoric triamide. The above-mentioned organic solvent can be used in a mixture of two or more types. Among them, aprotic organic solvents are desirable.

Reaction temperature is not particularly specified and the reaction can be carried out at various temperatures; for example, temperature range from 10 to 200 °C; in particular, 35 - 130 °C is more desirable. It is desirable to carry out the reaction under pressure, in particular, with ammonia pressurized at 0.3 - 2.5 kg/cm$^2$. The reaction is completed in 30 minutes to 24 hours, in general in about 1 - 10 hours.

The compounds of the formulae (3) and (4) can be used at the proper molar ratio; for example, 0.8 - 1.5 mols, particularly 1.0 - 1.2 mols of the compound of the formula (4) to 1.0 mol of the compound of the formula (3) is used desirably. Ammonia is used usually in excess to the compounds of the formulae (3) and (4).

To increase the yield of the desired compound, the above-mentioned reaction is preferably carried out in the presence of ammonium salt such as ammonium carbonate, ammonium chloride, ammonium sulfate, ammonium nitrate, ammonium phosphate, and ammonium acetate.

The above-mentioned ammonium salt can be used in an adequate amount, but it is desirable to use 0.05 - 1.0 mol of the ammonium salt to 1 mol of the compound of the formula (4).

The ester compound of the formula (1) which is obtained through the process of this invention can be converted to corresponding pyridine-2,3-dicarboxylic acid compound by hydrolysis using the known method. The hydrolysis can be carried out in water or aqueous solvent by the conventional method using basic compounds such as alkali metal hydroxides including sodium hydroxide and potassium hydroxide; alkali metal carbonates such as sodium carbonate and potassium carbonate; and alkali metal hydrogen carbonates such as sodium hydrogencarbonate and potassium hydrogencarbonate, however, alkali metal hydroxides are desirable to achieve completely the reaction. The hydrolysis is carried out at room temperature to 150 °C, preferably at 40 - 100 °C and completed usually in about 1 - 24 hours.

The desired carboxylic acid compounds can be obtained by subjecting the carboxylic acid salts produced by the above hydrolysis to acid precipitation with a mineral acid such as hydrochloric acid, sulfuric acid, nitric acid, or phosphoric acid at 20 - 60 °C.

In the aforementioned reaction scheme 1 the compounds of the formula (2) which are starting materials are known compounds. As the process to prepare the compound of the formula (3), the method described in J. Amer., Chem., Soc., Vol. 72, 5221, (1950) is known, but the method described therein, namely the compound of the formula (2) having ethyl group for $R^2$ and sodium for M is reacted with sulfuryl chloride or bromine in chloroform provides the object compound in the yield of as low as 31%, which is not suited to manufacturing the compound of the formula (3) in the industrial scale. According to the process of the invention, since the compound of the formula (2) is reacted with acid and halogenating agent, the

compound of the formula (3) can be obtained in a high yield in contrast to the above method, resulting in improvement of the yield of the compound of the formula (1).

Industrial Applicability

Pyridine-2,3-dicarboxylic acid compounds obtained by the process of this invention is useful as the intermediate for synthesizing various compounds such as agricultural chemicals and pharmaceuticals.

The process to prepare pyridine-2,3-dicarboxylic acid compounds according to the invention can produce the pyridine-2,3-dicarboxylic acid compounds with less steps and in high yield. In particular, it has a feature to carry out the reaction without isolating the intermediate. Further, the process permits the use of inexpensive readily available starting material, can be performed in safety because the reaction proceeds under the mild conditions, and realizes easy disposal of waste liquid. Thus, the pyridine-2,3-dicarboxylic acid compounds can be manufactured in the industrial scale.

Examples

Hereinafter, the invention is described in detail with reference to Manufacturing example and Examples, but it should be understood that the invention is not limited to these examples alone.

Manufacturing example 1

In a 300-ml four-neck flask were placed 41 g of dimethylamine hydrochloride (0.502 mol) and 42.7 g of 37% formalin (0.527 mol), and 84 g of n-decylaldehyde (0.527 mol) was added dropwise thereto at 20 to 30°C over a period of about 1 hour. After allowing to react for 6 hours at 70 to 75°C, the mixture was heated for 30 minutes at 110°C. Cooling down to room temperature, the solution was separated into oil layer and water layer. The oil layer (88 g) was distilled in vacuo, and 69 g of 2-(n-octyl)-2-propenal ($bp_{15}$: 108.5 to 110°C) was obtained.
IR (liquid film): 2930, 2850, 1700 $cm^{-1}$.

Example 1

In a 500-ml four-neck flask with a reflux condenser were placed 221 ml of chloroform and 13.1 g of 90% formic acid (0.26 mol), and 44.2 g of oxalacetic acid diethyl ester sodium salt (0.21 mol) was added thereto at room temperature. After stirring for 2 hours at 20 to 25°C, 41.1 g of sulfuryl chloride (0.30 mol) was added dropwise thereto at 20 to 30°C over a period of about 2 hours, and the mixture was allowed to react for 5 hours at 40°C. After cooling the content to room temperature, it was degassed in vacuo, and the resultant inorganic salt was filtered off. The filtrate was analyzed by gas chromatography, and α-chlorooxalacetic acid diethyl ester was obtained in a yield of 78%.

Next, this filtrate and 17.7 g of 2-ethyl-2-propenal (0.21 mol) were mixed in a 100-ml glass autoclave. After closing the autoclave, the internal temperature was raised to 110°C, and ammonia gas was introduced until the ammonia partial pressure reached 0.5 to 2.5 kg/$cm^2$, and the reaction was continued for 5 hours. The content was cooled to room temperature, and the insoluble matter was filtered off. The filtrate was concentrated, and the residue was distilled, and 5-ethyl-2,3-diethoxycarbonylpyridine ($bp_2$: 151 to 152°C) was obtained in a yield of 62.4% based on the charged oxalacetic acid diethyl ester sodium salt.

This reaction was performed by using various solvents, acids and halogenating agents. The types of solvents, acids and halogenating agents and yields of the desired compound (by gas chromatography analysis) are shown in Table 1.

Table 1

| Solvent | Type of acid | Type of halogenating agent | Yield of diethyl α-chlorooxalacetate (%) | Yield of 5-ethyl-2,3-diethoxycarbonyl pyridine (%) |
|---|---|---|---|---|
| Chloroform | Hydrochloric acid | Sulfuryl chloride | 54.5 | 41.4 |
| Chloroform | Acetic acid | Sulfuryl chloride | 41.7 | 30.0 |
| Methylene chloride | Formic acid | Sulfuryl chloride | 65.3 | 50.0 |
| Benzene | Formic acid | Sulfuryl chloride | 40.1 | 24.0 |
| Toluene | Formic acid | Sulfuryl chloride | 64.5 | 48.5 |
| Toluene | Formic acid | Chlorine | 46.9 | 29.0 |

The yield shows the amount based on the charged oxalacetic acid diethyl ester sodium salt.

Example 2

In a 200-liter glass lining vessel with reflux condenser, 104 kg of chloroform, 4.7 kg of 88% formic acid (89.9 mols) and 1.4 kg of ethanol were mixed at room temperature, and 14.1 kg of oxalacetic acid diethyl ester sodium salt (67.1 mols) was added thereto at 20 to 25 °C. The mixture was stirred for 2 hours at 10 to

25°C, and 11.4 kg of sulfuryl chloride (84.5 mols) was added dropwise at 10°C to 25°C over a period of about 2 hours. After stirring for 3 hours at 40°C, the mixture was refluxed for 2 hours at 60°C, and the gas produced by the reaction was purged out. After cooling the content to room temperature, 30 liters of water was added to dissolve the inorganic salt produced by the reaction, and the mixture was separated into chloroform layer and water layer. The separated 120.6 kg of chloroform solution was analyzed by gas chromatography, and 12.3 kg of α-chlorooxalacetic acid diethyl ester (55.2 mols) was obtained. This is a yield of 82.3% based on the charged oxalacetic acid diethyl ester sodium salt.

Next, this chloroform solution, 5.6 kg of 2-ethyl-2-propenal (66.6 mols) and 1.0 kg of ammonium acetate (13.0 mols) were mixed in a 100-liter glass autoclave. After closing it, the internal temperature was raised up to 60°C to discharge the air in the vapor phase, and the temperature was further raised to 105°C, and ammonia gas was introduced thereto until the internal pressure reached 3.5 to 6.0 kg/cm$^2$ and the reaction was conducted for 8 hours. After cooling the content to room temperature, 16 liters of water was added to dissolve the inorganic salt produced by the reaction to separate into the chloroform layer and water layer. The separated chloroform layer was concentrated, and 17.4 kg of brown oily matter was obtained. This concentrate was distilled, and 12.0 kg of 5-ethyl-2,3-diethoxycarbonylpyridine (47.8 mols) was obtained. This is a yield of 71.2% based on the charged oxalacetic acid diethyl ester sodium salt, and a yield of 86.6% based on the chlorinated compound.

Example 3

In a 300-ml four-neck flask, 130 ml of toluene, 21 g of 35% hydrochloric acid, and 40 g of water were mixed, and with vigorously stirring at 20 to 25°C in nitrogen atmosphere, 40 g of oxalacetic acid diethyl ester sodium salt (0.19 mol) was added. After stirring for 2 hours, the solution was let stand still to be separated into toluene layer and water layer. The toluene layer was washed with 100 ml of water, and was dried overnight over anhydrous sodium sulfate, and filtered. The filtrate was kept at 10 to 20°C, and 17 g of sulfuryl chloride (0.126 mol) was added dropwise over a period of 1 hour, and the reaction was continued for 5 hours at 40°C. After cooling the content to room temperature, it was degassed in vacuo. The obtained solution was analyzed by gas chromatography, and α-chlorooxalacetic acid diethyl ester was obtained in a yield of 64% based on the charged oxalacetic acid diethyl ester sodium salt.

Next, in a 300-ml glass autoclave, the obtained solution, 8.2 g of 2-methyl-2-propenal (0.117 mol) and 1.8 g of ammonium acetate (0.023 mol) were mixed. After closing the autoclave, the internal temperature was raised up to 110°C, and ammonia gas was introduced thereto so that the internal pressure be 1.5 kg/cm$^2$ or less, and the reaction was continued for 7 hours at 105 to 110°C. After cooling the content to room temperature, the insoluble matter was filtered off. The obtained filtrate was analyzed by gas chromatography, and 5-methyl-2,3-diethoxycarbonylpyridine was obtained in a yield of 42.6% based on the charged oxalacetic acid diethyl ester sodium salt.

To this obtained solution, 50 g of water and 61.6 g of 48% sodium hydroxide aqueous solution were added, and the solution was stirred under nitrogen atmosphere for 8.5 hours at 85 to 88°C. After termination of reaction, the reaction mixture was separated into toluene layer and water layer, and the water layer was decolored with activated carbon, and was treated with 50% sulfuric acid, and white crystals of 5-methylpyridine-2,3-dicarboxylic acid (mp: 184 to 186°C) were obtained. The recrystallized product from methanol-acetone mixed solvent had a melting point of 187 to 188.5°C (decomposition).

| Element analysis: As $C_8N_7NO_4$, | | | |
|---|---|---|---|
| | C | H | N |
| Calc'd. | 53.06 | 3.90 | 7.73 |
| Found | 52.63 | 3.95 | 7.68 |

NMR($CDCl_3$)ppm : 2.45 (3H, s), 8.15 (1H, s), 8.57 (1H, s)

Example 4

In a 1-liter four-neck flask, 450 ml of toluene, 65 ml of 35% hydrochloric acid and 150 ml of water were added, and the mixture was vigorously stirred in nitrogen atmosphere, while 143 g of oxalacetic acid diethyl ester sodium salt (0.68 mol) was added at room temperature. After stirring for 2 hours at 20 to 25°C, it was separated into toluene layer and water layer. The toluene layer was sequentially washed with dilute sodium

hydroxide aqueous solution and water, and was dried overnight over anhydrous sodium sulfate, and was filtered. While keeping the filtrate at 10 to 20 °C, 87.2 g of sulfuryl chloride (0.65 mol) was added dropwise over a period of about 2.5 hours. Afterwards, the internal temperature was raised to 45 °C to allow to react for 3 hours. The content was cooled to room temperature, and was degassed in vacuo. The obtained solution was analyzed by gas chromatography, and α-chlorooxalacetic acid diethyl ester was obtained in a yield of 65% based on the charged oxalacetic acid diethyl ester sodium salt.

Next, in a 1-liter glass autoclave, the obtained solution and 78.9 g of 2-(n-octyl)-2-propenal (0.47 mol) were mixed. After closing the autoclave, the internal temperature was raised to 110 °C, and the ammonia gas was introduced thereto so that the internal pressure be 0.8 to 2.0 kg/cm$^2$, and the reaction was continued for 10 hours at 110 to 115 °C. After cooling the content to room temperature, the insoluble matter was filtered off, and the filtrate was sequentially washed with 70 ml of 0.3N hydrochloric acid aqueous solution, and 50 ml of water. To this toluene solution, 107 g of 48% sodium hydroxide aqueous solution and 168 ml of water were added, and the mixture was refluxed at 85 to 87 °C for 2 hours under nitrogen atmosphere. After termination of the reaction, the mixture was diluted with 300 ml of hot water, and was separated into water layer and toluene layer. The water layer was treated with 12% hydrochloric acid aqueous solution at 70 to 80 °C, and 26 g of white plate-shaped crystals of 5-(n-octyl)pyridine-2,3-dicarboxylic acid (mp: 156 to 158 °C) were obtained. When recrystallized from ethanol-water mixed solvent, the melting point was 160.5 to 162 °C.

IR(KBr) : 3040, 2900, 2835, 1700 - 1600, 1560 cm$^{-1}$

| Element analysis: As $C_{15}N_{21}NO_4$, | | | |
|---|---|---|---|
| | C | H | N |
| Calc'd. | 64.51 | 7.58 | 5.01 |
| Found | 64.63 | 7.52 | 5.32 |

NMR($CDCl_3$)ppm : 0.95 (3H, t), 1.26 - 3.0 (14H, m), 9.15 (1H, s), 9.20 (1H, s)

Example 5

To a 500-ml four-neck flask, 210 ml of chloroform and 14.0 g of 90% formic acid (0.274 mol) were added, and 44.2 g of oxalacetic acid diethyl ester sodium salt (0.21 mol) was added thereto at room temperature. After stirring for 2 hours at 20 to 25 °C, 40 g of bromine (0.25 mol) was added dropwise over a period of 30 minutes at 10 to 15 °C, and after allowing to react for 3 hours at 30 to 40 °C and for 1 hour at 60 °C, the content was cooled to room temperature, and the resultant inorganic salt was filtered off.

Next, in a 100-ml glass autoclave, the obtained filtrate, 15 g of 2-ethyl-2-propenal (0.178 mol) and 3.2 g of ammonium acetate were mixed. After closing the autoclave, the internal temperature was raised to 110 °C, and ammonia gas was introduced thereto so that the internal pressure be 2.5 to 3.0 kg/cm$^2$, and the reaction was continued for 9.5 hours. After cooling the content to room temperature, the insoluble matter was filtered off, and the filtrate was analyzed by gas chromatography, and 5-ethyl-2,3-diethoxycarbonylpyridine was obtained in a yield of 30% based on the charged oxalacetic acid diethyl ester sodium salt.

## Claims

**1.** A process for preparing pyridine-2,3-dicarboxylic acid compounds represented by the formula (1):

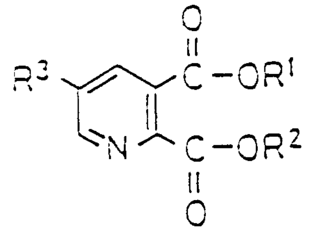

(1)

wherein R1 and R2 are, identical or different, a lower alkyl group, and R3 is a hydrogen atom or a lower

alkyl group, which comprises,
reacting the compound of the formula (2):

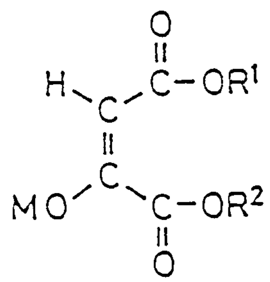

(2)

wherein R1 and R2 are the same as defined above and M is an alkali metal, with an acid and a halogenating agent to give the compound of the formula (3):

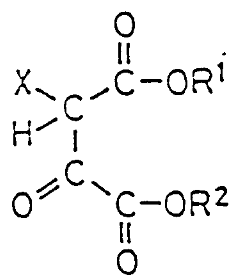

(3)

wherein R1 and R2 are the same as defined above and X is a halogen atom,
and then reacting the resultant product with the compound of the formula (4):

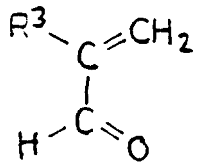

(4)

wherein R3 is the same as defined above, and ammonia,

wherein the compound of formula (3) resulting from the reaction of the compound of formula (2) with an acid and a halogenating agent is reacted without isolation with the compound of formula (4) and ammonia.

**2.** A process for preparing pyridine-2,3-dicarboxylic acid compounds according to Claim 1, in which the reaction of the compound of the formula (2) with a halogenating agent is carried out in an aprotic organic solvent and in the presence of an acid at a temperature of 0 - 100 °C.

**3.** A process for preparing pyridine-2,3-dicarboxylic acid compounds according to Claim 2, in which the aprotic organic solvent is chloroform, the acid is formic acid, and the halogenating agent is sulfuryl chloride.

**4.** A process for preparing pyridine-2,3-dicarboxylic acid compounds according to Claim 3, in which the reaction of the compounds of the formulae (3) and (4) and ammonia is carried out under pressure and at a temperature of 10 - 200 °C.

**5.** A process for preparing pyridine-2,3-dicarboxylic acid compounds according to any of Claims 1 to 4, in which the reaction of the compounds of the formulae (3) and (4) and ammonia is carried out in the

presence of an ammonium salt.

**Patentansprüche**

**1.** Verfahren zur Herstellung von Pyridin-2,3-dicarbonsäure-Verbindungen der Formel (1):

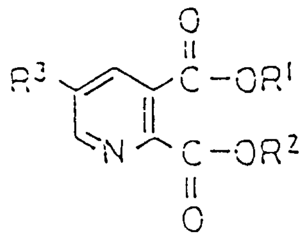

(1)

worin $R^1$ und $R^2$, gleich oder verschieden, eine Niederalkyl-Gruppe sind und $R^3$ ein Wasserstoffatom oder eine Niederalkyl-Gruppe ist,
welches umfaßt:
Umsetzung der Verbindung der Formel (2):

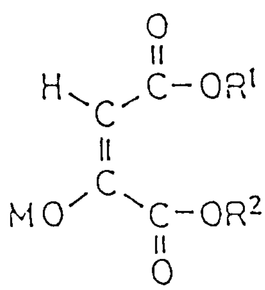

(2)

worin $R^1$ und $R^2$ wie oben definiert sind und M ein Alkalimetall ist, mit einer Säure und einem Halogenierungsmittel zur Bildung der Verbindung der Formel (3):

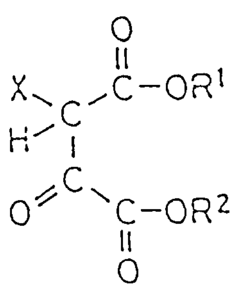

(3)

worin $R^1$ und $R^2$ wie oben definiert sind und X ein Halogenatom ist, und dann Umsetzung des resultierenden Produkts mit der Verbindung der Formel (4):

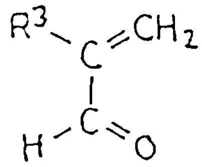

(4)

worin $R^3$ wie oben definiert ist, und Ammoniak, wobei die Verbindung der Formel (3), die aus der Reaktion der Verbindung der Formel (2) mit einer Säure und einem Halogenierungsmittel resultiert, ohne Isolierung mit der Verbindung der Formel (4) und Ammoniak umgesetzt wird.

**2.** Verfahren zur Herstellung von Pyridin-2,3-dicarbonsäure-Verbindungen gemäß Anspruch 1, worin die Reaktion der Verbindung der Formel (2) mit einem Halogenierungsmittel in einem aprotischen organischen Lösungsmittel und in Gegenwart einer Säure bei einer Temperatur von 0 bis 100 ° C ausgeführt wird.

**3.** Verfahren zur Herstellung von Pyridin-2,3-dicarbonsäure-Verbindungen gemäß Anspruch 2, worin das aprotische organische Lösungsmittel Chloroform ist, die Säure Ameisensäure ist und das Halogenierungsmittel Sulfurylchlorid ist.

**4.** Verfahren zur Herstellung von Pyridin-2,3-dicarbonsäure-Verbindungen gemäß Anspruch 3, worin die Reaktion der Verbindungen der Formeln (3) und (4) und Ammoniak unter Druck und bei einer Temperatur von 10 bis 200 ° C ausgeführt wird.

**5.** Verfahren zur Herstellung von Pyridin-2,3-dicarbonsäure-Verbindungen gemäß einem der Ansprüche 1 bis 4, worin die Reaktion der Verbindungen der Formeln (3) und (4) und Ammoniak in Gegenwart eines Ammoniumsalzes ausgeführt wird.

## Revendications

**1.** Procédé de préparation de composés de type acide pyridine-2,3-dicarboxylique représentés par la formule (1) :

(1)

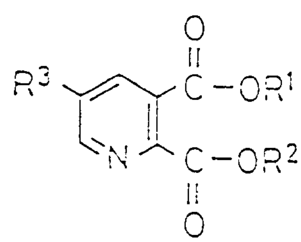

dans laquelle $R^1$ et $R^2$, identiques ou différents, représentent un groupe alkyle inférieur et $R^3$ est un atome d'hydrogène ou un groupe alkyle inférieur, le procédé comprenant la réaction d'un composé de formule (2) :

(2)

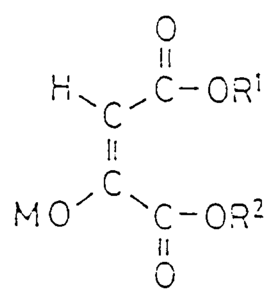

dans laquelle $R^1$ et $R^2$ sont identiques et ont la signification indiquée ci-dessus et M est un métal alcalin,
avec un acide et un agent d'halogénation pour obtenir le composé de formule (3) :

(3) 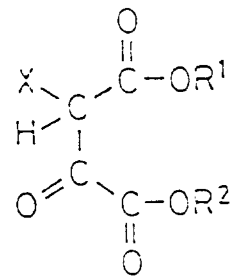

dans laquelle $R^1$ et $R^2$ sont identiques et ont la signification indiquée ci-dessus et X représente un atome d'halogène, puis
la réaction du composé de formule (3) ci-dessus avec un composé de formule (4) :

(4) 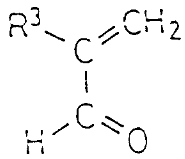

dans laquelle $R^3$ a la signification indiquée ci-dessus, et avec de l'ammoniac,
procédé dans lequel on fait réagir le composé de formule (3) obtenu par la réaction du composé de formule (2) avec un acide et un agent d'halogénation, sans étape d'isolement, avec le composé de formule (4) et de l'ammoniac.

**2.** Procédé de préparation de composés de type acide pyridine-2,3-dicarboxylique conforme à la revendication 1 dans lequel la réaction du composé de formule (2) avec un agent d'halogénation est réalisée dans un solvant organique aprotique et en présence d'un acide à une température comprise entre 0 et 100 °C.

**3.** Procédé de préparation de composés de type acide pyridine-2,3-dicarboxylique conforme à la revendication 2 dans lequel le solvant organique aprotique est le chloroforme, l'acide est l'acide formique et l'agent d'halogénation est le chlorure de sulfuryle.

**4.** Procédé de préparation de composés de type acide pyridine-2,3-dicarboxyliquesconforme à la revendication 3 dans lequel la réaction entre les composés de formule (3) et (4) et l'ammoniac est réalisée sous pression et à une température comprise entre 10 et 200 °C.

**5.** Procédé de préparation de composés de type acide pyridine-2,3-dicarboxylique conforme à une quelconque des revendications 1 à 4 dans lequel on réalise la réaction entre les composés de formule (3) et (4) et l'ammoniac en présence d'un sel d'ammonium.